# EUROPEAN PATENT APPLICATION

(11) **EP 4 381 959 A1**
(43) Date of publication of application: **12.06.2024**
(21) Application number: 22852790.9
(22) Date of filing: 11.07.2022
(51) Int. Cl.: A23L 2/38, A23L 2/00, A23L 2/52, C12N 1/20

(54) **PACKAGED BEVERAGE HAVING SUPPRESSED OFFENSIVE TASTE AND ODOR ASSOCIATED WITH LACTIC ACID BACTERIA, AND METHOD FOR PRODUCING SAME**

(30) Priority: 03.08.2021 JP 2021127443
(71) Applicant: Kirin Beverage Company, Limited, Tokyo 101-8645 (JP)
(72) Inventor: KITAZAWA, Ayumi, Tokyo 164-0001 (JP); MIYAMOTO, Kano, Tokyo 164-0001 (JP)
(74) Representative: Hoffmann Eitle
(86) International application number: PCT/JP2022/027217
(87) International publication number: WO 2023/013364

(57) **Abstract**

An object of the present invention is to provide, for example, a packaged beverage having suppressed offensive taste and odor specific to lactic acid bacteria, and a method for producing the same, preferably, to provide, for example, a packaged beverage having suppressed offensive taste and odor specific to lactic acid bacteria with the flavor balance maintained, and a method for producing the same. Provided is a method for producing a packaged beverage comprising 0.03% by weight or more of a lactic acid bacterium with respect to the total amount of the beverage, the method comprising the step of: including one or more compounds selected from the group consisting of limonene, citral, and octanal in the beverage.

## Description

### Technical Field

The present invention relates to a packaged beverage having suppressed offensive taste and odor associated with lactic acid bacteria, and a method for producing the same.

### Background Art

Foods and beverages having health-promoting effect have been recently attracting more and more attention because of the increase in health consciousness, and, for example, needs for fermented milks containing lactic acid bacteria and beverages containing lactic acid bacteria have been growing. However, beverages containing lactic acid bacteria suffer from a problem of a feeling of conspicuous offensive taste and odor specific to lactic acid bacteria in some cases.

As a method to solve the problem, for example, Patent Document 1 (Japanese unexamined Patent Application Publication No. 2013-94154) describes a method for producing a lactic acid bacteria beverage that gives a fresh feeling as a thirst-quenching beverage and has an improved taste as a lactic acid bacteria beverage with the offensive taste and odor derived from lactic acid bacteria suppressed by setting the ratio of the number of cells of lactic acid bacteria to the nonfat milk solid content (% by mass) to a specific ratio and including a specific galactomannan in the lactic acid bacteria beverage. However, inclusion of a galactomannan tends to result in deterioration of the refreshing feeling, and hence another solution that can solve the aforementioned problem has been demanded.

Known as fragrance components of citrus fruits such as lemon are, for example, limonene, citral, γ-terpinene, octanal, linalool, geraniol, and nerol.

However, it has not been known that offensive taste and odor specific to lactic acid bacteria in a packaged beverage containing a lactic acid bacterium can be suppressed with the flavor balance of the packaged beverage maintained by including one or more fragrance components selected from the group consisting of limonene, citral, and octanal in the packaged beverage at specific concentrations.

### Prior Art Document

### Patent Document

Patent Document 1: Japanese unexamined Patent Application Publication No. 2013-94154

### Summary of the Invention

### Object to be Solved by the Invention

An object of the present invention is to provide, for example, a packaged beverage having suppressed offensive taste and odor specific to lactic acid bacteria, and a method for producing the same, preferably, to provide, for example, a packaged beverage having suppressed offensive taste and odor specific to lactic acid bacteria with the flavor balance maintained, and a method for producing the same.

### Means to Solve the Object

The present inventors diligently examined to solve the object and found matters of (A) to (C) below, and eventually completed the present invention.
(A) Offensive taste and odor specific to lactic acid bacteria (culture odor and rough texture) are felt from a packaged beverage containing a lactic acid bacterium if the loading rate of the lactic acid bacterium (in terms of a dried product) is 0.003% by weight or more with respect to the total amount of the beverage.
(B) Offensive taste and odor specific to lactic acid bacteria (culture odor and rough texture) in a packaged beverage containing a lactic acid bacterium are suppressed by the sweetness of a sweetening component if the sweetening component is contained at 1.2% by weight or more as a sucrose-based sweetness level; on the other hand, offensive taste and odor specific to lactic acid bacteria (culture odor and rough texture) are felt if the sweetening component is contained at 1.0% by weight or less as a sucrose-based sweetness level.
(C) Offensive taste and odor specific to lactic acid bacteria (culture odor and rough texture) in a packaged beverage containing a lactic acid bacterium are successfully suppressed, preferably, offensive taste and odor specific to lactic acid bacteria (culture odor and rough texture) are successfully suppressed with the flavor balance of the packaged beverage maintained thereby, by including one or more fragrance components selected from the group consisting of limonene, citral, and octanal (hereinafter, each also referred to as a "fragrance component in the present invention") in the beverage at specific concentrations.

For example, the present invention specifically relates to:
(1) a packaged beverage comprising 0.003% by weight or more of a lactic acid bacterium with respect to a total amount of the beverage, wherein the beverage comprises one or more compounds selected from the group consisting of limonene, citral, and octanal;
(2) the packaged beverage according to the (1), comprising one or more compounds selected from the group consisting of (a) 500 to 15000 ppb of limonene, (b) 300 to 7000 ppb of citral, and (c) 30 to 100 ppb of octanal;
(3) the packaged beverage according to the (1) or (2), comprising:
   500 to 10000 ppb of limonene and 300 to 5000 ppb of citral; or
   500 to 10000 ppb of limonene and 30 to 100 ppb of octanal;
(4) the packaged beverage according to the (1) or (2), comprising:
   500 to 5000 ppb of limonene and 3000 to 5000 ppb of citral; or
   500 to 5000 ppb of limonene and 30 to 100 ppb of octanal;
(5) the packaged beverage according to any one of the (1) to (4), wherein the lactic acid bacterium is a bacterium of the genus *Lactococcus;*
(6) the packaged beverage according to any of the (1) to (5), wherein the lactic acid bacterium is *Lactococcus lactis* subsp. *lactis* JCM5805 strain.
(7) the packaged beverage according to any one of the (1) to (6), wherein
   (x) the beverage comprises no sweetening component, or
   (y) the beverage comprises a sweetening component and a content thereof is 1% by weight or less as a sucrose-based sweetness level;
(8) a method for producing a packaged beverage comprising 0.003% by weight or more of a lactic acid bacterium with respect to a total amount of the beverage, the method comprising the step of: including one or more compounds selected from the group consisting of limonene, citral, and octanal in the beverage; and
(9) a method for suppressing offensive taste and odor caused by a lactic acid bacterium in a packaged beverage comprising 0.003% by weight or more of a lactic acid bacterium with respect to a total amount of the beverage, the method comprising the step of: including one or more compounds selected from the group consisting of limonene, citral, and octanal in the beverage, in the production of the packaged beverage.

### Effect of the Invention

The present invention can provide, for example, a packaged beverage having suppressed offensive taste and odor specific to lactic acid bacteria, and a method for producing the same, and can preferably provide, for example, a packaged beverage having suppressed offensive taste and odor specific to lactic acid bacteria with the flavor balance maintained, and a method for producing the same.

### Brief Description of Drawing

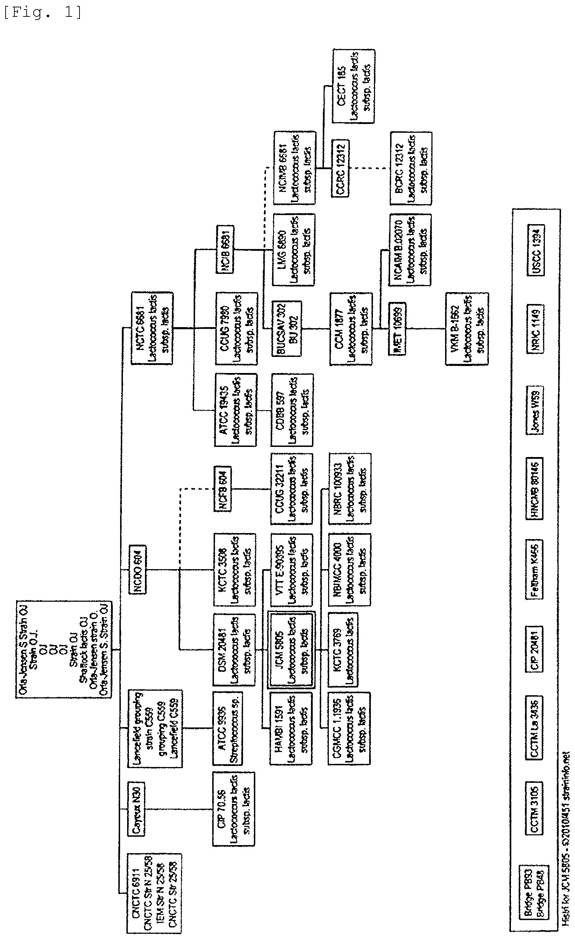

[Figure 1] Figure 1 is a diagram illustrating the relationship between *Lactococcus lactis* subsp. *lactis* JCM5805 strain and equivalent strains to that strain (strains derived from that strain and strains from which that strain is derived).

### Mode of Carrying Out the Invention

The present invention includes the following embodiments:
[1] a packaged beverage comprising 0.003% by weight or more of a lactic acid bacterium with respect to a total amount of the beverage, wherein the beverage comprises one or more compounds selected from the group consisting of limonene, citral, and octanal;
[2] a method for producing a packaged beverage comprising 0.003% by weight or more of a lactic acid bacterium with respect to a total amount of the beverage, the method comprising the step of: including one or more compounds selected from the group consisting of limonene, citral, and octanal in the beverage; and
[3] a method for suppressing offensive taste and odor caused by a lactic acid bacterium in a packaged beverage comprising 0.003% by weight or more of a lactic acid bacterium with respect to a total amount of the beverage, the method comprising the step of: including one or more compounds selected from the group consisting of limonene, citral, and octanal in the beverage, in the production of the packaged beverage.

### (Packaged Beverage)

The packaged beverage of the present invention is a packaged beverage comprising 0.003% by weight or more of a lactic acid bacterium with respect to a total amount of the beverage, wherein the beverage comprises one or more compounds selected from the group consisting of limonene, citral, and octanal (fragrance components in the present invention).

The "beverage" of the present invention is not limited as long as the beverage contains 0.003% by weight or more of a lactic acid bacterium with respect to the total amount of the beverage and contains one or more compounds selected from the group consisting of limonene, citral, and octanal, and preferable examples thereof include a functional beverage, a non-lactic beverage, a sports beverage, a near-water drink, and a carbonated beverage. Sports beverages are beverages that contain much minerals and are drunk primarily for the purpose of quickly supplying water and minerals lost as sweat through exercise or the like. Near-water drinks include drinks looking like water at first glance, beverages to which turbidity has been imparted with an emulsifying agent, and beverages supplemented with a sweetening component or a fragrance. It is preferable that the beverage of the present invention be an alcohol-free beverage.

### (One or More Fragrance Components Selected from the Group Consisting of Limonene, Citral, and Octanal)

An example of fragrance components in the present invention is one or more compounds selected from the group consisting of limonene, citral, and octanal, and, especially, a preferable example thereof is one or more compounds selected from the group consisting of (a) 500 to 15000 ppb of limonene, (b) 300 to 7000 ppb of citral, and (c) 30 to 100 ppb of octanal.

In the case that limonene is used in the present invention, a preferable example of the limonene concentration in the beverage from the viewpoints of degree of suppression of offensive taste and odor specific to lactic acid bacteria and harmonization with the flavor balance of the packaged beverage is 500 to 15000 ppb or 700 to 15000 ppb or 1000 to 15000 ppb, preferably 5000 to 15000 ppb, and more preferably 10000 to 15000 ppb. In the case that citral and/or octanal are/is used in addition to limonene, citral and/or octanal preferably each at a concentration in a range that does not largely deteriorate the flavor balance of the packaged beverage can be further used in addition to limonene at any of the limonene concentrations shown above. Each limonene concentration shown above is a particularly preferable example of the limonene concentration in the beverage in the case that limonene is used alone.

In the case that citral is used in the present invention, a preferable example of the citral concentration in the beverage from the viewpoints of degree of suppression of offensive taste and odor specific to lactic acid bacteria and harmonization with the flavor balance of the packaged beverage is 300 to 7000 ppb or 500 to 7000 ppb, preferably 1000 to 7000 ppb or 3000 to 7000 ppb, and more preferably 5000 to 7000 ppb. In the case that limonene and/or octanal are/is used in addition to citral, limonene and/or octanal preferably each at a concentration in a range that does not largely deteriorate the flavor balance of the packaged beverage can be further used in addition to citral at any of the citral concentrations shown above. Each citral concentration shown above is a particularly preferable example of the citral concentration in the beverage in the case that citral is used alone.

In the case that octanal is used in the present invention, a preferable example of the octanal concentration in the beverage from the viewpoints of degree of suppression of offensive taste and odor specific to lactic acid bacteria and harmonization with the flavor balance of the packaged beverage is 30 to 100 ppb, and preferably 50 to 100 ppb. In the case that limonene and/or citral are/is used in addition to octanal, limonene and/or citral preferably each at a concentration in a range that does not largely deteriorate the flavor balance of the packaged beverage can be further used in addition to octanal at any of the octanal concentrations shown above. Each octanal concentration shown above is a particularly preferable example of the octanal concentration in the beverage in the case that octanal is used alone.

In the case that two or more compounds selected from the group consisting of limonene, citral, and octanal are used as fragrance components in the present invention, the concentrations are, for example:
500 to 15000 ppb of limonene and more than 0 ppb to 7000 ppb of citral;
more than 0 ppb to 15000 ppb of limonene and 300 to 7000 ppb of citral;
500 to 15000 ppb of limonene and more than 0 ppb to 100 ppb of octanal;
more than 0 ppb to 15000 ppb of limonene and 30 to 100 ppb of octanal;
300 to 7000 ppb of citral and more than 0 ppb to 100 ppb of octanal; or
more than 0 ppb to 7000 ppb of citral and 30 to 100 ppb of octanal.

In the case that at least limonene and citral are used (e.g., in the case that limonene and citral are used and octanal is not) in the present invention, the limonene and citral concentrations in the beverage are, for example, 500 to 15000 ppb of limonene and more than 0 ppb to 7000 ppb of citral; or more than 0 ppb to 15000 ppb of limonene and 300 to 7000 ppb of citral, preferably, for example, 500 to 10000 ppb of limonene and 300 to 5000 ppb of citral from the viewpoints of degree of suppression of offensive taste and odor specific to lactic acid bacteria and harmonization with the flavor balance of the packaged beverage, and particularly preferably, for example, 500 to 5000 ppb of limonene and 3000 to 5000 ppb of citral for the availability of additional synergistic effect.

In the case that octanal is used in addition to limonene and citral, octanal preferably at a concentration in a range that does not largely deteriorate the flavor balance of the packaged beverage can be further used in addition to limonene at any of the limonene concentrations shown above and citral at any of the citral concentrations shown above.

In the case that at least limonene and octanal are used (e.g., in the case that limonene and octanal are used and citral is not) in the present invention, the limonene and octanal concentrations in the beverage are, for example, 500 to 15000 ppb of limonene and more than 0 ppb to 100 ppb of octanal; or more than 0 ppb to 15000 ppb of limonene and 30 to 100 ppb of octanal, preferably, for example, 500 to 10000 ppb of limonene and 30 to 100 ppb of octanal from the viewpoints of degree of suppression of offensive taste and odor specific to lactic acid bacteria and harmonization with the flavor balance of the packaged beverage, and particularly preferably, for example, 500 to 5000 ppb of limonene and 30 to 100 ppb of octanal for the availability of additional synergistic effect.

In the case that citral is used in addition to limonene and octanal, citral preferably at a concentration in a range that does not largely deteriorate the flavor balance of the packaged beverage can be further used in addition to limonene at any of the limonene concentrations shown above and octanal at any of the octanal concentrations shown above.

In the case that at least citral and octanal are used (e.g., in the case that citral and octanal are used and limonene is not) in the present invention, the citral and octanal concentrations in the beverage are, for example, 300 to 7000 ppb of citral and more than 0 ppb to 100 ppb of octanal; or more than 0 ppb to 7000 ppb of citral and 30 to 100 ppb of octanal. In the case that limonene is used in addition to citral and octanal, limonene preferably at a concentration in a range that does not largely deteriorate the flavor balance of the packaged beverage can be further used in addition to citral at any of the citral concentrations shown above and octanal at any of the octanal concentrations shown above.

Concentration adjustment for fragrance components in the present invention in the packaged beverage of the present invention can be performed, for example, by adjusting the amounts of fragrance components in the present invention to be contained in (or added to) the packaged beverage.

The limonene, citral, and octanal concentrations in the packaged beverage of the present invention can be measured by gas chromatography-mass spectrometry (GC-MS method).

### (Lactic Acid Bacterium)

The packaged beverage of the present invention contains a lactic acid bacterium.

The lactic acid bacterium to be used in producing the packaged beverage of the present invention may be any lactic acid bacterium without limitation, and may be viable cells of a lactic acid bacterium or dead cells of a lactic acid bacterium; however, it is preferable that the lactic acid bacterium be dead cells of a lactic acid bacterium. The dead cells of a lactic acid bacterium may be a dried product or non-dried product; however, from the viewpoint of storage stability or the like, the lactic acid bacterium is preferably, for example, a dried product of a lactic acid bacterium, and more preferably, for example, a dried powder of a lactic acid bacterium.

The formation of a dry powder is not limited to a particular method, and a dry powder can be formed through such treatment as freeze-drying, hot-air drying, or spray drying; however, powder formation by spray drying is preferred.

Even if viable cells of a lactic acid bacterium are used in producing the packaged beverage of the present invention, most of the cells of the lactic acid bacterium fundamentally become dead cells through a step of heat-sterilizing.

The term "lactic acid bacterium" is a collective term indicative of any organism taxonomically certified to be a lactic acid bacterium, and not limited by the genus, species, strain, and so on. Examples of the "lactic acid bacterium" include a bacterium that produces large amounts of lactic acid (preferably, lactic acid in an amount of 50% or more of the amount of saccharide consumed) through lactic acid fermentation of saccharide, such as a bacterium of the genus *Lactobacillus,* a bacterium of the genus *Streptococcus,* a bacterium of the genus *Lactococcus,* a bacterium of the genus *Leuconostoc,* a bacterium of the genus *Pediococcus,* and a bacterium of the genus *Enterococcus.*

The genus and species of the lactic acid bacterium to be used in the present invention are not limited, and the lactic acid bacterium is, for example, one or more bacteria selected from the group consisting of bacteria of the genus *Lactobacillus,* bacteria of the genus *Streptococcus,* bacteria of the genus *Lactococcus,* bacteria of the genus *Leuconostoc,* bacteria of the genus *Pediococcus,* and bacteria of the genus *Enterococcus,* preferably, for example, one or more bacteria selected from the group consisting of bacteria of the genus *Lactococcus,* and more preferably, for example, one or more bacteria selected from the group consisting of *Lactococcus lactis* subsp. *lactis* (hereinafter, also referred to as *"Lactococcus lactis",* simply).

A more specific, preferred mode of the lactic acid bacterium in the present invention is, for example, one or more bacteria selected from the group consisting of *Lactobacillus acidophilus, Lactobacillus delbrueckii* subsp. *bulgaricus, Lactobacillus delbrueckii* subsp. *delbrueckii, Lactobacillus delbrueckii* subsp. *lactis, Lactobacillus casei, Lactobacillus paracasei, Lactobacillus gasseri, Lactobacillus helveticus, Lactobacillus johnsonii, Lactobacillus plantarum, Lactobacillus brevis, Lactobacillus casei* subsp. *rhamnosus, Lactobacillus pentosus, Lactobacillus fermentum, Streptococcus salivarius* subsp. *thermophilus, Lactococcus lactis* subsp. *lactis, Lactococcus lactis* subsp. *lactis* biovar *diacetylactis, Lactococcus lactis* subsp. *cremoris, Lactococcus raffinolactis, Lactococcus piscium, Lactococcus plantarum, Lactococcus garvieae, Lactococcus lactis* subsp. *hordniae, Leuconostoc mesenteroides* subsp. *cremoris, Leuconostoc lactis, Pediococcus damnosus, Pediococcus pentosaceus, Pediococcus acidilactici, Enterococcus faecalis,* and *Enterococcus faecium;* preferably, for example, one or more bacteria selected from the group consisting of *Lactobacillus acidophilus, Lactobacillus delbrueckii* subsp. *bulgaricus, Lactobacillus delbrueckii* subsp. *lactis, Lactobacillus delbrueckii* subsp. *delbrueckii, Lactobacillus casei, Lactobacillus paracasei, Lactobacillus gasseri, Lactobacillus helveticus, Lactobacillus johnsonii, Lactobacillus plantarum, Lactobacillus brevis, Lactobacillus casei* subsp. *rhamnosus, Lactobacillus pentosus, Lactobacillus fermentum, Streptococcus salivarius* subsp. *thermophilus, Lactococcus lactis* subsp. *lactis, Lactococcus lactis* subsp. *lactis* biovar *diacetylactis, Lactococcus lactis* subsp. *cremoris, Lactococcus raffinolactis, Lactococcus piscium, Lactococcus plantarum, Lactococcus garvieae, Lactococcus lactis* subsp. *hordniae, Leuconostoc mesenteroides* subsp. *cremoris,* and *Leuconostoc lactis;* more preferably, for example, one or more bacteria selected from the group consisting of *Lactococcus lactis, Lactococcus lactis* subsp. *lactis* biovar *diacetylactis, Lactococcus lactis* subsp. *cremoris, Lactococcus raffinolactis, Lactococcus piscium, Lactococcus plantarum, Lactococcus garvieae,* and *Lactococcus lactis* subsp. *hordniae;* even more preferably, for example, *Lactococcus lactis;* furthermore preferably, for example, one or more bacteria selected from the group consisting of *Lactococcus lactis* JCM5805, *Lactococcus lactis* JCM20101, *Lactococcus lactis* NBRC12007, and *Lactococcus lactis* NRIC1150; and particularly preferably, for example, *Lactococcus lactis* JCM5805. A preferred mode of the *Lactococcus lactis* JCM5805 strain is preferably dead cells of JCM5805 strain, more preferably a dried product of dead cells of JCM5805 strain, even more preferably a dried powder of dead cells of JCM5805 strain, and furthermore preferably a spray-dried powder of dead cells of JCM5805 strain.

Any equivalent bacterial strain to JCM5805 strain is included in the category of the bacterial strain "JCM5805 strain" in the present invention as long as the effect of the present invention is achieved. Here, the equivalent bacterial strain refers to a bacterial strain derived from JCM5805 strain (bacterial strain A), a bacterial strain from which JCM5805 strain is derived (bacterial strain B), or a progeny bacterial strain of the bacterial strain A or bacterial strain B. Such equivalent bacterial strains may be preserved in other culture collection institutes. Figure 1 shows bacterial strains derived from JCM5805 strain and bacterial strains from which JCM5805 strain is derived. Any of the equivalent bacterial strains to JCM5805 strain in Figure 1 can also be used as "JCM5805 strain" in the present invention, as long as the effect of the present invention is achieved.

The lactic acid bacterium for use in the present invention is available from depositary institutions and the like such as American Type Culture Collection (the United States). A commercially available starter culture may be used as the lactic acid bacterium for use in the present invention.

The preparation of the lactic acid bacterium for use in the present invention is not limited to a particular method, and examples of methods for preparing viable cells of a lactic acid bacterium can include a method of collecting bacterial cells from medium in which a lactic acid bacterium has been cultured through filtration, centrifugation, or the like. Examples of methods for preparing dead cells of a lactic acid bacterium include a method of sterilizing medium in which a lactic acid bacterium has been cultured and then collecting bacterial cells therefrom through filtration, centrifugation, or the like, and a method of collecting bacterial cells from medium in which a lactic acid bacterium has been cultured through filtration, centrifugation, or the like and then sterilizing, and drying treatment or crushing treatment can be additionally performed, as necessary.

The sterilization is not limited to a particular means, and not only heating, but also any common practice to kill bacteria such as ultraviolet or γ-ray irradiation can be applied.

In the present invention, the lactic acid bacterium content in the packaged beverage is not limited as long as the lactic acid bacterium content is 0.003% by weight or more with respect to the total amount of the beverage in terms of the dry weight of the lactic acid bacterium, and is preferably, for example, 0.005% by weight or more, 0.0075% by weight or more, 0.01% by weight or more, or more than 0.01% by weight, from the viewpoint that the present invention becomes more significant because of the resulting enhanced offensive taste and odor specific to lactic acid bacteria.

The upper limit of the lactic acid bacterium content is not limited, and is, for example, 0.2% by weight, 0.1% by weight, 0.07% by weight, or 0.05% by weight.

Those upper limit values and lower limit values can be arbitrarily combined.

In the case that the packaged beverage of the present invention contains two or more lactic acid bacteria, the above content is the total for all the lactic acid bacteria.

### (Optional Components)

To the packaged beverage of the present invention, beverage additives that are used in the production of common packaged beverages, for example, any one or more additives of acidulants, pigments, fruit juice, sweetening components (including high-intensity sweeteners), milk raw materials, and food additives (e.g., antioxidants, preservatives, thickening stabilizers, emulsifying agents, dietary fibers, pH-adjusting agents) may be added, or not. The packaged beverage of the present invention may be provided as a carbonated beverage through inclusion of carbon dioxide gas.

The packaged beverage of the present invention may contain a fragrance other than the one or more compounds selected from the group consisting of limonene, citral, and octanal, but it is preferable from the viewpoint of benefiting more from the significance of the present invention that the packaged beverage of the present invention be free of a fragrance other than the one or more compounds selected from the group consisting of limonene, citral, and octanal.

The packaged beverage of the present invention may contain an acidulant or not, but it is preferable for the packaged beverage of the present invention to contain an acidulant. The acidulant is, for example, one or more substances selected from citric acid, phosphoric acid, ascorbic acid, gluconic acid, succinic acid, tartaric acid, lactic acid, fumaric acid, malic acid, and phytic acid, and salts of them. Examples of the salts include a salt with an inorganic base and a salt with an organic base. Examples of the salt with an inorganic base include an alkali metal salt (e.g., a sodium salt, a potassium salt) and an ammonium salt. Examples of the salt with an organic base include an amine salt (e.g., a methylamine salt, a diethylamine salt, a triethylamine salt, an ethylenediamine salt) and an alkanolamine salt (e.g., a monoethanolamine salt, a diethanolamine salt, a triethanolamine salt). Preferable among them is the alkali metal salt, and specific examples thereof include trisodium citrate, monopotassium citrate, tripotassium citrate, sodium gluconate, potassium gluconate, sodium tartrate, trisodium tartrate, potassium hydrogen tartrate, sodium lactate, potassium lactate, and sodium fumarate.

The packaged beverage of the present invention may contain a milk raw material (i.e., a raw material containing a milk fat and/or a nonfat milk solid), but it is preferable from the viewpoint of benefiting more from the significance of the present invention that the packaged beverage of the present invention contain a milk raw material at low concentration or be free of a milk raw material.

Examples of the case that a milk raw material is contained at low concentration include the case that the milk fat content is more than 0% by weight and 0.1% by weight or less, more than 0% by weight and 0.05% by weight or less, or more than 0% by weight and 0.02% by weight or less with respect to the total amount of the beverage and/or the nonfat milk solid content is more than 0% by weight and 0.4% by weight or less, more than 0% by weight and 0.2% by weight or less, or more than 0% by weight and 0.08% by weight or less with respect to the total amount of the beverage.

### (Sweetening component)

The packaged beverage of the present invention may contain a sweetening component, but preferably, for example,
(x) the packaged beverage of the present invention contains no sweetening component, or
(y) the packaged beverage of the present invention contains a sweetening component, and the content thereof is 1% by weight or less as a sucrose-based sweetness level, from the viewpoint of benefiting more from the significance of the present invention.

The term "sweetening component" in the present invention refers to a component that can impart sweetness to foods and beverages, and, for example, sweetening components having a relative sweetness level of 0.05 or more are preferably included in the category. The term "sweetening component" in the present invention encompasses not only sweetening components that are commonly used as an additive for foods and beverages, but also sweetening components derived from fruit juice and so on (sucrose, glucose, fructose, etc.). Examples of sweetening components that are commonly used as an additive for foods and beverages can include: crystalline saccharide such as monosaccharide such as fructose, glucose, tagatose, and arabinose, disaccharide such as lactose, trehalose, maltose, and sucrose, and polysaccharide such as powdered starch syrup; and non-crystalline saccharide such as oligosaccharide such as maltooligosaccharide and galactooligosaccharide, starch syrup, and isomerized sugar syrup (e.g., high-fructose corn syrup). Additional examples can include sugar alcohol such as maltitol, lactitol, sorbitol, mannitol, xylitol, and erythritol. Still additional examples can include a high-intensity sweetening component (high-intensity sweetener) such as sucralose, *Stevia rebaudiana* extract, licorice root extract, thaumatin, glycyrrhizin, saccharin, aspartame, and acesulfame K.

A "sucrose-based sweetness level (% by weight)" in the present invention is a numerical value calculated from the following expression. Sweetness level = sweetening component content (g/100 g) × relative sweetness level of that sweetening component

The "sweetening component content (g/100 g)" indicates the amount (g) of a sweetening component contained per 100 g of the beverage of the present invention, i.e., concentration (g/100 g), and the relative sweetness level indicates the relative intensity of sweetness of a sweetening component of certain type as the sweetness of sucrose at 20°C is assumed as 1. Accordingly, the "sweetness level" in the present invention indicates the sweetening component concentration (g/100 g) on the basis of sucrose, and reflects the degree of sweetness as a feeling felt by a person who has ingested the beverage of the present invention. In the case that two or more sweetening components are contained in the beverage of the present invention, a value of "sweetening component content (g/100 g) × relative sweetness level" is calculated for each sweetening component, and the sum total of the calculated values is defined as the sweetness level of the beverage of the present invention. The sweetening component content of the beverage of the present invention can be measured by applying a known method such as an HPLC method, a GC-MS method, and an LC-MS method to the beverage.

The relative sweetness levels of various sweetening components are known, and the following shows examples thereof.
Glucose (0.7), fructose (0.6), maltose (0.4), sorbitol (0.7), maltitol (0.8), *Stevia rebaudiana* extract (150), glycyrrhizin (170), acesulfame K (200), aspartame (200), saccharin (300), sucralose (600), alitame (2000), thaumatin (3000).

As an example of calculation of sweetness levels in the present invention, determination of the sweetness level of the beverage of the present invention containing 0.001 g of glucose (relative sweetness level: 0.7) and 0.00001 g of sucralose (relative sweetness level: 600) will be shown. In this case, the sweetness level in the present invention is given as the sum total of a numerical value calculated from 0.001 × 0.7 ("0.0007") and a numerical value calculated from 0.000001 × 600 ("0.0006"), and the result is 0.0013% by weight.

### (Production of the Packaged beverage of the Present Invention)

The packaged beverage of the present invention can be produced by a method for producing a packaged beverage comprising 0.003% by weight or more of a lactic acid bacterium with respect to a total amount of the beverage, the method comprising the step of: including one or more compounds selected from the group consisting of limonene, citral, and octanal in the beverage. Any common method for producing a packaged beverage containing lactic acid bacteria can be applied to the production method of the present invention for parts other than setting the lactic acid bacterium content to 0.003% by weight or more and including one or more compounds selected from the group consisting of limonene, citral, and octanal in packaged beverage. Examples of such common production methods include a method of producing a packaged beverage through the steps of blending, packing, and heat-sterilizing. In producing the packaged beverage of the present invention, any beverage additive that is used for formulation design of common packaged beverages may be added, and the timing of adding the additive is not limited. For more specific information on the aforementioned common method for producing a packaged beverage, for example, reference can be made to "Soft Drinks - Newly Revised Edition (KORIN K. K.)".

Preferable examples of the "step of including a lactic acid bacterium" include adding a lactic acid bacterium so that the beverage can contain the lactic acid bacterium, including not only adding a lactic acid bacterium to a blend solution itself but also adding a lactic acid bacterium to water to be used for preparing a blend solution.

In the present invention, the timing of including (preferably adding) a lactic acid bacterium may be any phase of the production process for the packaged beverage as long as the effect of the present invention is achieved, but is preferably any phase before heat sterilization. Specifically, for example, a lactic acid bacterium may be included in a blend solution or water at the step of blending; alternatively, a lactic acid bacterium may be included in a blend solution immediately before or immediately after the step of packing in a package.

### (Heat Sterilization)

It is preferable that the packaged beverage of the present invention be a packaged beverage that has been heat-sterilized. The step of heat-sterilizing may be before or after packing the beverage in a package.

Various heat sterilization methods that are commonly used in the field of foods can be used for the heat sterilization, and examples thereof include a method with a hot water spray type, hot water storage type, or steam type retort sterilizer, or a tube type or plate type liquid continuous sterilizer.

For the heat sterilization in the present invention, common conditions that are used for heat sterilization of packaged beverages can be used without limitation. Examples of such conditions include conditions that give an F₀ value of 4 to 40, and preferred in that relatively short time suffices for sterilization are, for example, conditions of 111 to 150°C and a time that gives an F₀ value of 4 to 20.

### (Package)

The beverage of the present invention is a packaged beverage. The term package means a sealed package capable of preventing its content and the air from coming into contact, and examples thereof include a metal can, a barrel, a plastic bottle (e.g., a PET bottle or cup), a paper package, a bottle, and a pouch.

### (Packaged Beverage Having Suppressed Offensive Taste and Odor Specific to Lactic Acid Bacteria)

The packaged beverage of the present invention is a packaged beverage having suppressed offensive taste and odor specific to lactic acid bacteria. Herein, the phrase, the packaged beverage "having suppressed offensive taste and odor specific to lactic acid bacteria", means a packaged beverage having suppressed offensive taste and odor specific to lactic acid bacteria (i.e., culture odor and/or rough texture) as compared with a packaged beverage produced by the same production method with raw materials of the same types except that one or more compounds selected from the group consisting of limonene, citral, and octanal (hereinafter, also referred to as "the control beverage in the present invention") is not included in the packaged beverage. A preferable example of the packaged beverage "having suppressed offensive taste and odor specific to lactic acid bacteria" in the present specification is a packaged beverage that shows, when evaluated on the basis of evaluation criteria in Table 1 shown later in Examples, an average point (preferably an average point rounded to the first decimal place) of grades of 3.5 to 7 points (i.e., the rating in Table 2 shown later in Examples is "fair", "good", or "very good"), and a more preferable example thereof is a packaged beverage that shows 4.5 to 7 points (the rating in Table 2 is "good" or "very good").

### (Packaged Beverage with Flavor Balance Maintained)

It is preferable that the packaged beverage of the present invention not only have suppressed offensive taste and odor specific to lactic acid but also have the flavor balance maintained. Herein, the phrase, the packaged beverage "with the flavor balance maintained", means that the one or more compounds selected from the group consisting of limonene, citral, and octanal cause no excessively strong flavor and thus no excessively uncomfortable feeling, and acceptable flavor disharmony results. A preferable example of the packaged beverage "with the flavor balance maintained" is a packaged beverage that is rated as "good" or "fair" when evaluated with an evaluation method in Table 3 shown later in Examples (an evaluation method using evaluation criteria in Table 5), and a more preferable example thereof is a packaged beverage that is rated as "good".

### (Method for Suppressing Offensive Taste and Odor Caused by Lactic Acid Bacterium)

The method of the present invention for suppressing offensive taste and odor caused by a lactic acid bacterium is not limited as long as the method is a method for suppressing offensive taste and odor caused by a lactic acid bacterium in a packaged beverage comprising 0.003% by weight or more of a lactic acid bacterium with respect to a total amount of the beverage, the method comprising the step of: including one or more compounds selected from the group consisting of limonene, citral, and octanal in the beverage, in the production of the packaged beverage.

Hereinafter, the present invention will be described in detail with Examples; however, the present invention is not limited to those Examples.

### Examples

### [Test 1] Influence on Flavor of Beverage by Including Lactic Acid Bacterium in Beverage

A test shown below was carried out to examine whether including a lactic acid bacterium in a beverage has influence on the flavor of the beverage, and if so, how is the influence on the flavor of the beverage depending on the amounts of the lactic acid bacterium included.

### (Preparation of Beverage Base)

To water, 0.01% by weight of 85% phosphoric acid and trisodium citrate (crystal) were added and then mixed together to give a beverage base.

### (Preparation of Sample Beverages)

A dried dead cell powder of the lactic acid bacterium *Lactococcus lactis* subsp. *lactis* JCM5805 strain was added to the beverage base at a loading rate (% by weight) shown later in Table 3, and then mixed together; thus, sample beverages of Test Examples 1 to 7 were prepared.

### (Sensory Evaluation for Sample Beverages)

Six trained sensory evaluators performed sensory evaluation on offensive taste and odor specific to lactic acid bacteria for the sample beverages on the basis of evaluation criteria shown in Table 1. In sensory evaluation of offensive taste and odor specific to lactic acid bacteria for a sample beverage, an average point (an average point rounded to the first decimal place) of grades given by the six sensory evaluators was calculated, and the average point of grades was applied to criteria in Table 2 to determine the rating of the sample beverage on offensive taste and odor.

**[Table 1]**

| Grade | Evaluation criteria for offensive taste and odor specific to lactic acid bacteria | |
|---|---|---|
| 7 | Offensive taste and odor specific to lactic acid bacteria (culture odor and rough texture) were | not felt at all |
| 6 | | hardly felt |
| 5 | | slightly felt |
| 4 | | felt to some extent |
| 3 | | felt |
| 2 | | strongly felt |
| 1 | | very strongly felt |

**[Table 2]**

| Rating | | Average grade |
|---|---|---|
| Pass | very good | 6.5 points or more and 7 points or less |
| | very good | 5.5 points or more and less than 6.5 points |
| | good | 4.5 points or more and less than 5.5 points |
| | fair | 3.5 points or more and less than 4.5 points |
| Fail | poor | 2.5 points or more and less than 3.5 points |
| | poor | 1.5 points or more and less than 2.5 points |
| | poor | 1 point or more and less than 1.5 points |

Table 3 shows results of the sensory evaluation.

**[Table 3]**

| Test Example | Lactic acid bacterium loading rate % | | Grade and rating on offensive taste and odor specific to lactic acid bacteria | |
|---|---|---|---|---|
| 1 | | 0.001 | 5.8 | very good |
| 2 | | 0.003 | 3.8 | fair |
| 3 | | 0.004 | 3.7 | fair |
| 4 | | 0.005 | 3.3 | poor |
| 5 | | 0.01 | 2.5 | poor |
| 6 | | 0.1 | 1.5 | poor |

As can be seen from the results in Table 3, it was shown that offensive taste and odor specific to lactic acid bacteria were generated when the loading rate of the lactic acid bacterium was 0.003% or more. That is, it was demonstrated that the problem for the present invention occurs if the loading rate of a lactic acid bacterium is 0.003% or more.

### [Test 2] Influence on Offensive Taste and Odor Specific to Lactic Acid Bacteria by Including Sweetening component

A test shown below was carried out to examine how is the influence on the offensive taste and odor specific to lactic acid bacteria by including a sweetening component in a beverage containing a lactic acid bacterium.

### (Preparation of Sample Beverages)

To the beverage base in Test 1, 0.01% by weight of a dried dead cell powder of the lactic acid bacterium JCM5805 strain and a specific amount of a sweetening component as shown later in Table 4 were added, and then mixed together; thus, sample beverages of Test Examples 7 to 14 were prepared. Acesulfame K (acesulfame potassium) has sweetness 200 times higher than that of sucrose. Therefore, the sucrose-based sweetness levels of Test Examples 8, 10, 12, and 14 were almost equivalent to those of Test Examples 7, 9, 11, and 13, respectively.

### (Sensory Evaluation for Sample Beverages)

Six trained sensory evaluators performed sensory evaluation on offensive taste and odor specific to lactic acid bacteria for the sample beverages in the same manner as in Test 1. Table 4 shows results of the sensory evaluation.

**[Table 4]**

| Test Example | Lactic acid bacterium loading rate % | Sweetening component | Sweetening component loading rate % | Sucrose-based sweetness level % | Grade and rating on offensive taste and odor specific to lactic acid bacteria | |
|---|---|---|---|---|---|---|
| 7 | 0.01 | granulated sugar | 0.750 | 0.75 | 3.2 | poor |
| 8 | | acesulfame K | 0.0037 | 0.74 | 2.8 | poor |
| 9 | | granulated sugar | 1.00 | 1.0 | 3.3 | poor |
| 10 | | acesulfame K | 0.005 | 1.0 | 3.3 | poor |
| 11 | | granulated sugar | 1.2 | 1.2 | 3.8 | fair |
| 12 | | acesulfame K | 0.06 | 1.2 | 4.8 | good |
| 13 | | granulated sugar | 10.0 | 10 | 6.8 | very good |
| 14 | | acesulfame K | 0.05 | 10 | 5.7 | very good |

As can be seen from the results in Table 4, at a sucrose-based sweetness level of 1.2% by weight or more, the rating on offensive taste and odor specific to lactic acid bacteria was "fair" to "very good", indicating the occurrence of effect to suppress the offensive taste and odor, because of the sweetness of the sweetening component (Test Examples 11 to 14). In contrast to this, at a sucrose-based sweetness level of 1.0% by weight or less, the offensive taste and odor specific to lactic acid bacteria were not suppressed, and thus it was suggested that the problem for the present invention is still present in such a case. In subsequent Tests 3 to 7, no sweetening component was added, and evaluation was performed for sugar-free sample beverages.

### [Test 3] Evaluation of Suppression of Offensive Taste and Odor Specific to Lactic Acid Bacteria by Including Fragrance Component - Part 1

A test shown below was carried out to examine whether offensive taste and odor specific to lactic acid bacteria can be suppressed by including a specific fragrance component in a beverage containing a lactic acid bacterium.

### (Preparation of Sample Beverages)

To the beverage base in Test 1, 0.01% by weight of a dried dead cell powder of the lactic acid bacterium JCM5805 strain and a specific amount of a fragrance component (citral, limonene, or octanal) as shown later in Tables 7 to 9 were added, and then mixed together; thus, sample beverages of Test Examples 15 to 36 were prepared.

### (Sensory Evaluation for Sample Beverages)

Six trained sensory evaluators performed sensory evaluation on offensive taste and odor specific to lactic acid bacteria for the sample beverages in the same manner as in Test 1.

In addition, six trained sensory evaluators performed sensory evaluation on flavor harmony through the addition of a fragrance component on the basis of evaluation criteria in Table 5. In rating a sample beverage on flavor harmony in the sensory evaluation, the most dominant rating among ratings given by the six sensory evaluators was employed, and when equal numbers of different ratings were present, the worse was employed. When two or more sensory evaluators rated a sample beverage as "poor", the sample beverage was rated as "poor" irrespective of ratings given by the other four sensory evaluators.

**[Table 5]**

| Rating | | Evaluation criteria for flavor harmony |
|---|---|---|
| Pass | good | Natural flavor. No flavor unharmony was felt. |
| | fair | Flavor unharmony (uncomfortable feeling) was somewhat felt. |
| Fail | poor | Flavor unharmony (uncomfortable feeling) was felt. |

Furthermore, ratings on offensive taste and odor specific to lactic acid bacteria and ratings on flavor harmony were totally evaluated on the basis of evaluation criteria in Table 6.

**[Table 6]**

| Total rating | | Evaluation criteria for total rating |
|---|---|---|
| Pass | good | Rating on offensive taste and odor specific to lactic acid bacteria was "very good", "good", or "fair", and rating on flavor harmony was "good" or "fair". |
| Fail | poor | At least one of rating on offensive taste and odor specific to lactic acid bacteria and rating on flavor harmony was "poor". |

Tables 7 to 9 show results of the sensory evaluations for the sample beverages of Test Examples 15 to 36.

**[Table 7]**

| Test Example | Lactic acid bacterium loading rate % | Fragrance component | Amount of fragrance component (ppb) | Grade and rating on offensive taste and odor specific to lactic acid bacteria | | Rating on flavor harmony | Total rating |
|---|---|---|---|---|---|---|---|
| 15 | 0.01 | citral | 100 | 3.0 | poor | good | poor |
| 16 | | | 300 | 4.0 | fair | good | good |
| 17 | | | 500 | 4.0 | fair | good | good |
| 18 | | | 1000 | 5.0 | good | good | good |
| 19 | | | 3000 | 5.2 | good | fair | good |
| 20 | | | 5000 | 5.7 | very good | fair | good |
| 21 | | | 7000 | 6.0 | very good | fair | good |
| 22 | | | 10000 | 6.2 | very good | poor | poor |
| 23 | | | 20000 | 6.7 | very good | poor | poor |

**[Table 8]**

| Test Example | Lactic acid bacterium loading rate % | Fragrance component | Amount of fragrance component (ppb) | Grade and rating on offensive taste and odor specific to lactic acid bacteria | | Rating on flavor harmony | Total rating |
|---|---|---|---|---|---|---|---|
| 24 | 0.01 | limonene | 200 | 3.2 | poor | good | poor |
| 25 | | | 500 | 3.5 | fair | good | good |
| 26 | | | 700 | 4.0 | fair | good | good |
| 27 | | | 1000 | 4.0 | fair | good | good |
| 28 | | | 5000 | 4.8 | good | good | good |
| 29 | | | 10000 | 6.0 | very good | fair | good |
| 30 | | | 15000 | 6.0 | very good | fair | good |
| 31 | | | 20000 | 6.2 | very good | poor | poor |

**[Table 9]**

| Test Example | Lactic acid bacterium loading rate % | Fragrance component | Amount of fragrance component (ppb) | Grade and rating on offensive taste and odor specific to lactic acid bacteria | | Rating on flavor harmony | Total rating |
|---|---|---|---|---|---|---|---|
| 32 | 0.01 | octanal | 10 | 3.3 | poor | good | poor |
| 33 | | | 30 | 4.3 | fair | good | good |
| 34 | | | 50 | 5.2 | good | good | good |
| 35 | | | 100 | 5.7 | very good | fair | good |
| 36 | | | 300 | 6.3 | very good | poor | poor |

As can be seen from the results in Table 7, the offensive taste and odor specific to lactic acid bacteria were suppressed at a citral concentration of 300 ppb or more, and the offensive taste and odor specific to lactic acid bacteria were more largely suppressed as the citral concentration was increased. By contrast, the flavor of citral was excessively strong at a citral concentration of 10000 ppb or more, and the rating on flavor harmony was "poor" (Table 7) . In summary, it was found that citral concentrations of 300 ppb to 7000 ppb allow the offensive taste and odor specific to lactic acid bacteria to be suppressed with keeping a rating of "good" or "fair" on flavor harmony, resulting in a total rating of "good" (Table 7). It was demonstrated that preferable citral concentrations are 1000 ppb to 7000 ppb, and more preferable citral concentrations are 5000 ppb to 7000 ppb from the viewpoint of more suppressing offensive taste and odor specific to lactic acid bacteria.

As can be seen from the results in Table 8, the offensive taste and odor specific to lactic acid bacteria were suppressed at a limonene concentration of 500 ppb or more, and the offensive taste and odor specific to lactic acid bacteria were more largely suppressed as the limonene concentration was increased. By contrast, the flavor of limonene was excessively strong at a limonene concentration of 20000 ppb, and the rating on flavor harmony was "poor" (Table 8). In summary, it was found that limonene concentrations of 500 ppb to 15000 ppb allow the offensive taste and odor specific to lactic acid bacteria to be suppressed with keeping a rating of "good" or "fair" on flavor harmony, resulting in a total rating of "good" (Table 8). It was demonstrated that preferable limonene concentrations are 5000 ppb to 15000 ppb, and more preferable limonene concentrations are 10000 ppb to 15000 ppb from the viewpoint of more suppressing offensive taste and odor specific to lactic acid bacteria.

As can be seen from the results in Table 9, the offensive taste and odor specific to lactic acid bacteria were suppressed at an octanal concentration of 30 ppb or more, and the offensive taste and odor specific to lactic acid bacteria were more largely suppressed as the octanal concentration was increased. By contrast, the flavor of octanal was excessively strong at an octanal concentration of 300 ppb, and the rating on flavor harmony was "poor" (Table 9). In summary, it was found that octanal concentrations of 30 ppb to 100 ppb allow the offensive taste and odor specific to lactic acid bacteria to be suppressed with keeping a rating of "good" or "fair" on flavor harmony, resulting in a total rating of "good" (Table 9). It was demonstrated that preferable octanal concentrations are 50 ppb to 100 ppb from the viewpoint of more suppressing offensive taste and odor specific to lactic acid bacteria.

### [Test 4] Evaluation of Suppression of Offensive Taste and Odor Specific to Lactic Acid Bacteria by Including Fragrance Component - Part 2

A test shown below was carried out to examine whether offensive taste and odor specific to lactic acid bacteria can be suppressed by including a specific fragrance component in a beverage containing a lactic acid bacterium even if the loading rate of the lactic acid bacterium to the beverage was 0.1%.

### (Preparation of Sample Beverages)

To the beverage base in Test 1, 0.1% by weight of a dried dead cell powder of the lactic acid bacterium JCM5805 strain and a specific amount of a fragrance component (limonene or octanal) as shown later in Table 10 were added, and then mixed together; thus, sample beverages of Test Examples 37 and 38 were prepared.

### (Sensory Evaluation for Sample Beverages)

Six trained sensory evaluators performed sensory evaluation on offensive taste and odor specific to lactic acid bacteria for the sample beverages in the same manner as in Test 1.

**[Table 10]**

| Test Example | Lactic acid bacterium loading rate % | Fragrance component | Amount of fragrance component (PPb) | Grade and rating on offensive taste and odor specific to lactic acid bacteria | | Rating on flavor harmony | Total rating |
|---|---|---|---|---|---|---|---|
| 37 | 0.1 | citral | 5000 | 4.7 | good | good | good |
| 38 | | octanal | 50 | 4.2 | fair | good | good |

Comparing the results for Test Examples 37 and 38 in Table 10 with the result for Test Example 6 (lactic acid bacterium loading rate: 0.1%, no fragrance component was added) in Table 3 (grade on offensive taste and odor specific to lactic acid bacteria: 1.5 points, rating: "poor") found that an improved grade and rating on offensive taste and odor specific to lactic acid bacteria were given to any of Test Examples 37 and 38; thus, it was found that offensive taste and odor specific to lactic acid bacteria can be suppressed by including a specific fragrance component in a beverage containing a lactic acid bacterium even if the loading rate of the lactic acid bacterium to the beverage was 0.1%.

### [Test 5] Evaluation of Suppression of Offensive Taste and Odor Specific to Lactic Acid Bacteria by Including Fragrance Component - Part 3

A test shown below was carried out to examine whether offensive taste and odor specific to lactic acid bacteria can be suppressed by including two specific fragrance components (limonene and citral) in a beverage containing a lactic acid bacterium, and whether any synergistic effect can be obtained by using the two fragrance components.

### (Preparation of Sample Beverages)

To the beverage base in Test 1, 0.01% by weight of a dried dead cell powder of the lactic acid bacterium JCM5805 strain and specific amounts of two fragrance components (limonene and citral) as shown later in Tables 12 to 15 were added, and then mixed together; thus, sample beverages of Test Examples 39 to 58 were prepared.

### (Sensory Evaluation for Sample Beverages)

Six trained sensory evaluators performed sensory evaluation on offensive taste and odor specific to lactic acid bacteria for the sample beverages in the same manner as in Test 1. Evaluation was performed on synergistic effect through the use of the two fragrance components on the basis of evaluation criteria in Table 11.

**[Table 11]**

| Rating on synergistic effect | Evaluation criteria for synergistic effect |
|---|---|
| ↑ | The grade on offensive taste and odor specific to lactic acid bacteria was 0.5 points or more higher than that for any of the two fragrance components singly used |
| → | The grade on offensive taste and odor specific to lactic acid bacteria was the same as or less than 0.5 points higher than that for any of the two fragrance components used singly |
| ↓ | The grade on offensive taste and odor specific to lactic acid bacteria was lower than that for at least either one of the two fragrance components used singly |

In addition, six trained sensory evaluators performed sensory evaluation on flavor harmony through the addition of the fragrance components in the same manner as in Test 3.

Furthermore, total evaluation was performed for the sample beverages in the same manner as in Test 3.

Tables 12 to 15 show results of the sensory evaluations and results of the evaluation on synergistic effect for the sample beverages of Tests Examples 39 to 58.

**[Table 12]**

| Test Example | Lactic acid bacterium loading rate % | Limonene (PPb) | Citral (ppb) | Grade and rating on offensive taste and odor specific to lactic acid bacteria | | Rating on flavor harmony | Total rating | Synergistic effect |
|---|---|---|---|---|---|---|---|---|
| 39 | 0.01 | 500 | 0 | 3.5 | fair | good | good | - |
| 40 | | | 300 | 4.0 | fair | good | good | → |
| 41 | | | 1000 | 4.7 | good | good | good | ↓ |
| 42 | | | 3000 | 5.8 | very good | fair | good | ↑ |
| 43 | | | 5000 | 6.3 | very good | fair | good | ↑ |

**[Table 13]**

| Test Example | Lactic acid bacterium loading rate % | Limonene (ppb) | Citral (PPb) | Grade and rating on offensive taste and odor specific to lactic acid bacteria | | Rating on flavor harmony | Total rating | Synergistic effect |
|---|---|---|---|---|---|---|---|---|
| 44 | 0.01 | 1000 | 0 | 4.0 | fair | good | good | - |
| 45 | | | 300 | 4.2 | fair | good | good | → |
| 46 | | | 1000 | 4.7 | good | good | good | ↓ |
| 47 | | | 3000 | 5.8 | very good | good | good | ↑ |
| 48 | | | 5000 | 6.2 | very good | fair | good | ↑ |

**[Table 14]**

| Test Example | Lactic acid bacterium loading rate % | Limonene (ppb) | Citral (ppb) | Grade and rating on offensive taste and odor specific to lactic acid bacteria | | Rating on flavor harmony | Total rating | Synergistic effect |
|---|---|---|---|---|---|---|---|---|
| 49 | 0.01 | 5000 | 0 | 4.8 | good | good | good | - |
| 50 | | | 300 | 5.0 | good | good | good | → |
| 51 | | | 1000 | 5.3 | good | good | good | → |
| 52 | | | 3000 | 6.0 | very good | fair | good | ↑ |
| 53 | | | 5000 | 6.5 | very good | fair | good | ↑ |

**[Table 15]**

| Test Example | Lactic acid bacterium loading rate % | Limonene (ppb) | Citral (ppb) | Grade and rating on offensive taste and odor specific to lactic acid bacteria | | Rating on flavor harmony | Total rating | Synergistic effect |
|---|---|---|---|---|---|---|---|---|
| 54 | 0.01 | 10000 | 0 | 6.0 | very good | good | good | - |
| 55 | | | 300 | 5.8 | very good | good | good | ↓ |
| 56 | | | 1000 | 5.7 | very good | good | good | ↓ |
| 57 | | | 3000 | 6.3 | very good | fair | good | → |
| 58 | | | 5000 | 6.2 | very good | fair | good | → |

As can be seen from the results in Tables 12 to 15, limonene concentrations in the range of 500 to 10000 ppb in combination with citral concentrations in the range of 300 to 5000 ppb allowed the offensive taste and odor specific to lactic acid bacteria to be suppressed, and gave a rating of "good" or "fair" on flavor harmony and thus a total rating of "good" in all cases. These results demonstrated that when the two fragrance components, limonene and citral, are used in combination, limonene concentrations in the range of 500 to 10000 ppb in combination with citral concentrations in the range of more than 0 ppb to 5000 ppb give a beverage with a total rating of "good".

The result of the evaluation on synergistic effect for each Test Example is a result given by comparing with a grade on offensive taste and odor specific to lactic acid bacteria for limonene alone (3.5 points for 500 ppb; 4.0 points for 1000 ppb; 4.8 points for 5000 ppb; or 6.0 points for 10000 ppb) and a grade on offensive taste and odor specific to lactic acid bacteria for citral alone (4.0 points for 300 ppb; 5.0 points for 1000 ppb; 5.2 points for 3000 ppb; or 5.7 points for 5000 ppb) on the basis of the evaluation criteria shown above in Table 11. For example, the grade on offensive taste and odor specific to lactic acid bacteria for Test Example 42, 5.8 points, was 0.5 points or more higher than any of the grade for limonene alone at 500 ppb, 3.5 points, and the grade for citral alone at 3000 ppb, 5.2 points, and hence Test Example 42 was rated as having synergistic effect (↑).

It was found from the results of the evaluation on synergistic effect in Tables 12 to 15 that when the two fragrance components, limonene and citral, were used in combination, limonene concentrations in the range of 500 to 5000 ppb in combination with citral concentrations in the range of 3000 to 5000 ppb gave synergistic effect to the effects to suppress offensive taste and odor specific to lactic acid bacteria. This effect is a significant effect. Those ranges also gave a total rating of "good", and were demonstrated to be preferable for the beverage of the present invention.

### [Test 6] Evaluation of Suppression of Offensive Taste and Odor Specific to Lactic Acid Bacteria by Including Fragrance Component - Part 4

A test shown below was carried out to examine whether offensive taste and odor specific to lactic acid bacteria can be suppressed by including two specific fragrance components (limonene and octanal) in a beverage containing a lactic acid bacterium, and whether any synergistic effect can be obtained by using the two fragrance components.

### (Preparation of Sample Beverages)

To the beverage base in Test 1, 0.01% by weight of a dried dead cell powder of the lactic acid bacterium JCM5805 strain and specific amounts of two fragrance components (limonene and octanal) as shown later in Tables 16 to 19 were added, and then mixed together; thus, sample beverages of Test Examples 59 to 69 were prepared.

### (Sensory Evaluation for Sample Beverages)

Six trained sensory evaluators performed sensory evaluation on offensive taste and odor specific to lactic acid bacteria for the sample beverages in the same manner as in Test 1. Evaluation was performed on synergistic effect through the use of the two fragrance components in the same manner as in Test 5.

In addition, six trained sensory evaluators performed sensory evaluation on flavor harmony through the addition of the fragrance components in the same manner as in Test 3.

Furthermore, total evaluation was performed for the sample beverages in the same manner as in Test 3.

Tables 16 to 19 show results of the sensory evaluations and results of the evaluation on synergistic effect for the sample beverages of Tests Examples 59 to 69.

**[Table 16]**

| Test Example | Lactic acid bacterium loading rate % | Limonene (ppb) | Octanal (ppb) | Grade and rating on offensive taste and odor specific to lactic acid bacteria | | Rating on flavor harmony | Total rating | Synergistic effect |
|---|---|---|---|---|---|---|---|---|
| 59 | 0.01 | 500 | 0 | 3.5 | fair | good | good | - |
| 60 | | | 30 | 4.8 | good | good | good | ↑ |
| 61 | | | 100 | 6.3 | very good | fair | good | ↑ |

**[Table 17]**

| Test Example | Lactic acid bacterium loading rate % | Limonene (ppb) | Octanal (ppb) | Grade and rating on offensive taste and odor specific to lactic acid bacteria | | Rating on flavor harmony | Total rating | Synergistic effect |
|---|---|---|---|---|---|---|---|---|
| 62 | 0.01 | 1000 | 0 | 4.0 | fair | good | good | - |
| 63 | | | 30 | 5.2 | good | good | good | ↑ |
| 64 | | | 100 | 6.2 | very good | good | good | ↑ |

**[Table 18]**

| Test Example | Lactic acid bacterium loading rate % | Limonene (ppb) | Octanal (ppb) | Grade and rating on offensive taste and odor specific to lactic acid bacteria | | Rating on flavor harmony | Total rating | Synergistic effect |
|---|---|---|---|---|---|---|---|---|
| 65 | 0.01 | 5000 | 0 | 4.8 | good | good | good | - |
| 66 | | | 30 | 5.7 | very good | good | good | ↑ |
| 67 | | | 100 | 6.2 | very good | good | good | ↑ |

**[Table 19]**

| Test Example | Lactic acid bacterium loading rate % | Limonene (ppb) | Octanal (ppb) | Grade and rating on offensive taste and odor specific to lactic acid bacteria | | Rating on flavor harmony | Total rating | Synergistic effect |
|---|---|---|---|---|---|---|---|---|
| 68 | 0.01 | 10000 | 0 | 6.0 | very good | good | good | - |
| 69 | | | 30 | 4.3 | fair | good | good | ↓ |

As can be seen from the results in Tables 16 to 19, limonene concentrations in the range of 500 to 5000 ppb in combination with octanal concentrations in the range of 30 to 100, and a limonene concentration of 10000 ppb in combination with an octanal concentration of 30 ppb allowed the offensive taste and odor specific to lactic acid bacteria to be suppressed, and gave a rating of "good" on flavor harmony and thus a total rating of "good" in all cases. These results demonstrated that when the two fragrance components, limonene and octanal, are used in combination, limonene concentrations in the range of 500 to 5000 ppb in combination with octanal concentrations of more than 0 ppb to 100 ppb, and limonene concentrations in the range of 500 to 10000 ppb in combination with octanal concentrations of more than 0 ppb to 30 ppb give a beverage with a total rating of "good".

The result of the evaluation on synergistic effect for each Test Example is a result given by comparing with a grade on offensive taste and odor specific to lactic acid bacteria for limonene alone (3.5 points for 500 ppb; 4.0 points for 1000 ppb; 4.8 points for 5000 ppb; or 6.0 points for 10000 ppb) and a grade on offensive taste and odor specific to lactic acid bacteria for octanal alone (4.3 points for 30 ppb; or 5.7 points for 100 ppb) on the basis of the evaluation criteria shown above in Table 11. For example, the grade on offensive taste and odor specific to lactic acid bacteria for Test Example 60, 4.8 points, was 0.5 points or more higher than any of the grade for limonene alone at 500 ppb, 3.5 points, and the grade for octanal alone at 30 ppb, 4.3 points, and hence Test Example 60 was rated as having synergistic effect (↑).

It was found from the results of the evaluation on synergistic effect in Tables 16 to 19 that when the two fragrance components, limonene and octanal, were used in combination, limonene concentrations in the range of 500 to 5000 ppb in combination with octanal concentrations in the range of 30 to 100 ppb gave synergistic effect to the effects to suppress offensive taste and odor specific to lactic acid bacteria. This effect is a significant effect. Those ranges also gave a total rating of "good", and were demonstrated to be preferable for the beverage of the present invention.

### [Test 7]

A test shown below was carried out to examine whether offensive taste and odor specific to lactic acid bacteria can be suppressed by including a specific fragrance component in a beverage containing a lactic acid bacterium even if the beverage containing a lactic acid bacterium is a carbonated beverage.

### (Preparation of Sample Beverages)

To carbonated water, 0.01% by weight of a dried dead cell powder of the lactic acid bacterium JCM5805 strain and 5000 ppb of limonene were added, and then mixed together; thus, a sample beverage of Test Example 71 was prepared. In addition, a sample beverage of Test Example 70 was produced in the same manner except that limonene was not added.

### (Sensory Evaluation for Sample Beverages)

Six trained sensory evaluators performed sensory evaluation on offensive taste and odor specific to lactic acid bacteria for the sample beverages in the same manner as in Test 1.

In addition, six trained sensory evaluators performed sensory evaluation on flavor harmony through the addition of the fragrance component in the same manner as in Test 3.

Furthermore, total evaluation was performed for the sample beverages in the same manner as in Test 3.

Table 20 shows results of the sensory evaluations for the sample beverages of Tests Examples 70 and 71.

**[Table 20]**

| Test Example | Lactic acid bacterium loading rate % | Limonene (PPb) | Grade and rating on offensive taste and odor specific to lactic acid bacteria | | Rating on flavor harmony | Total rating |
|---|---|---|---|---|---|---|
| 70 | 0.01 | 0 | 3.2 | poor | - | poor |
| 71 | | 5000 | 5.7 | very good | good | good |

As can be seen from the results in Table 20, it was demonstrated that offensive taste and odor specific to lactic acid bacteria can be suppressed by including a specific fragrance component in a beverage containing a lactic acid bacterium even if the beverage containing a lactic acid bacterium is a carbonated beverage.

## Claims

1. A packaged beverage comprising 0.003% by weight or more of a lactic acid bacterium with respect to a total amount of the beverage, wherein the beverage comprises one or more compounds selected from the group consisting of limonene, citral, and octanal.

2. The packaged beverage according to claim 1, comprising one or more compounds selected from the group consisting of (a) 500 to 15000 ppb of limonene, (b) 300 to 7000 ppb of citral, and (c) 30 to 100 ppb of octanal.

3. The packaged beverage according to claim 1 or 2, comprising:
500 to 10000 ppb of limonene and 300 to 5000 ppb of citral; or
500 to 10000 ppb of limonene and 30 to 100 ppb of octanal.

4. The packaged beverage according to claim 1 or 2, comprising:
500 to 5000 ppb of limonene and 3000 to 5000 ppb of citral; or
500 to 5000 ppb of limonene and 30 to 100 ppb of octanal.

5. The packaged beverage according to any one of claims 1 to 4, wherein the lactic acid bacterium is a bacterium of the genus *Lactococcus.*

6. The packaged beverage according to any one of claims 1 to 5, wherein the lactic acid bacterium is *Lactococcus lactis* subsp. *lactis* JCM5805 strain.

7. The packaged beverage according to any one of claims 1 to 6, wherein
(x) the beverage comprises no sweetening component, or
(y) the beverage comprises a sweetening component and a content thereof is 1% by weight or less as a sucrose-based sweetness level.

8. A method for producing a packaged beverage comprising 0.003% by weight or more of a lactic acid bacterium with respect to a total amount of the beverage, the method comprising the step of including one or more compounds selected from the group consisting of limonene, citral, and octanal in the beverage.

9. A method for suppressing offensive taste and odor caused by a lactic acid bacterium in a packaged beverage comprising 0.003% by weight or more of a lactic acid bacterium with respect to a total amount of the beverage, the method comprising the step of including one or more compounds selected from the group consisting of limonene, citral, and octanal in the beverage, in the production of the packaged beverage.
